# EUROPÄISCHE PATENTSCHRIFT

(11) **EP 1 759 899 B1**
(45) Veröffentlichungstag und Bekanntmachung des Hinweises auf die Patenterteilung: **19.08.2009**
(21) Anmeldenummer: 06017728.4
(22) Anmeldetag: 25.08.2006
(51) Int. Cl.: B60H 3/00, A61L 9/00

(54) **Dosierbares Duftstoffgerät zur Anwendung in einem Kraftfahrzeug**
Dosable fragrance device for use in a motor vehicle
Dispositif dosable de fragrance utilisé dans un véhicule automobile

(30) Priorität: 05.09.2005 DE 102005041985
(43) Veröffentlichungstag der Anmeldung: 07.03.2007
(73) Patentinhaber: GM Global Technology Operations, Inc., Detroit, MI 48265-3000 (US)
(72) Erfinder: Schenk, Holger, 65205 Wiesbaden (DE)
(74) Vertreter: Strauss, Peter

(56) Entgegenhaltungen:
- EP-A- 1 479 400
- EP-A- 1 561 477
- DE-U1- 9 005 101
- DE-U1- 20 212 778
- DE-U1- 29 719 510
- DE-U1- 29 812 056
- DE-U1-5202004 010 01
- US-A1- 2004 007 787
- US-A1- 2004 067 729

## Beschreibung

Die Erfindung betrifft ein dosierbares Duftstoffgerät zur Anwendung in einem Kraftfahrzeug, insbesondere einem Personenkraftfahrzeug, mit einem im Fahrzeuginneren angeordneten Gehäuse zur Aufnahme eines Duftstoffes, wobei das Gehäuse mit mindestens einer Lufteintrittsöffnung und mindestens einer Luftaustrittsöffnung sowie einer Nachfüllöffnung für den Duftstoff versehen ist, und die mindestens eine Duftaustrittsöffnung durch Verstellen einer Abdeckung geöffnet und verschlossen werden kann.

Heutige Duftspender für Personenkraftfahrzeuge werden größtenteils über den Zubehörhandel vertrieben. Es gibt sie in Form von Behältnissen, die an die Lüftungsgitter eingeklipst werden. Weiterhin gibt es Duftspender, die als Granulat im Aschenbecher wirken oder als am Rückspiegel hängender "Duftbaum" Duftstoffe verbreiten.

Es sind ferner dosierbare Duftstoffgeräte zur Anwendung in Personenkraftfahrzeugen bekannt.

Ein dosierbares Duftstoffgerät der eingangs genannten Art ist aus der EP-A-1 479 400 bekannt. Bei diesem ist das Gehäuse durch ein Hauptteil und einen in diesen eingesteckten Boden gebildet. Der Boden dient der Aufnahme des Duftstoffs, der den vom Boden und dem Hauptteil umschlossenen Raum im Wesentlichen aufnimmt. Zum Nachfüllen des Duftstoffs wird der Boden vom Hauptteil getrennt und der Duftstoff in den Boden eingesetzt. Das Hauptteil ist länglich und nach oben zulaufend ausgebildet mit einem ebenen Wandungsteil und einem von diesen ausgehenden Kreissegmentwandungsteil. Das ebene Wandungsteil ist mit den Lufteintrittsöffnungen versehen und weist überdies Flügel zum Einstecken des Duftstoffgerätes in ein Lüftungsgitter eines Kraftfahrzeuges auf. Das Kreissegmentwandungsteil ist mit einer Vielzahl von Luftaustrittsöffnungen versehen. Auf das Hauptteil ist ein drehbares Element gesteckt, das in einem oberen Bereich das Hauptteil hintergreift und weiter unten einen Wandungsabschnitt aufweist, der entsprechend der Bogenform des Kreissegmentwandungsteils gebogen ist und Öffnungen aufweist, die in definierter Drehstellung des drehbaren Elements in fluchtender Anordnung mit den Luftaustrittsöffnungen des Kreissegmentwandungsteils bringbar sind. Verstellt wird das drehbare Element durch Ergreifen des Elements im oberen Bereich und Drehen.

Ein dosierbares Duftstoffgerät zur Anwendung in einem Kraftfahrzeug ist in der DE 202 12 778 U1 beschrieben. Das Duftstoffgerät ist integraler Bestandteil der Luftdüse des Fahrzeuges, um Duftstoffe direkt oder über einen Zuführungskanal in den Luftstrom im Innenraum des Gehäuses der Luftdüse wahlweise oder gesteuert abgeben zu können. Es weist eine Vielzahl von Luftaustrittsöffnungen auf, die so ausgebildet sind, dass eine Zerstäubung der Duftstoffe in den Innenraum der Luftdüse erfolgt. Durch den aus den Luftaustrittsöffnungen austretenden Luftstrom werden die Duftstoffe mit der Luft in dem Innenraum des Fahrzeugs beschleunigt vermischt und sie verteilen sich durch die Lüftströmung sehr schnell. Um ein Ausbringen des Duftstoffs zu verhindern, sind die Luftaustrittsöffnungen durch Verstellen einer Abdeckung verschließbar, diese Abdeckung ist beispielsweise als Klappe oder Scheibenverschluss ausgebildet.

In der DE 20 2004 010 015 U1 ist ein dosierbares Duftstoffgerät zur Anordnung in einem Personenkraftfahrzeug beschrieben, bei dem ein im Fahrzeuginneren angeordnetes Gehäuse eine um deren Längsachse drehbare Kartusche aufnimmt, in die einen Duftstoffträger eingesetzt ist. Dieser Duftstoffträger ist beispielsweise ein Feststoff oder Formkörper, der mit einem Duftöl oder Parfum getränkt ist. Das Gehäuse weist seitlich angeordnete Durchströmöffnungen auf, die mit einer Belüftungsöffnung in der Instrumententafel und dem Innenraum des Kraftfahrzeugs in Strömungsverbindung stehen. Die Kartusche hat eine mit der Durchströmöffnung im Gehäuse korrespondierende Abströmöffnung und ist so im Gehäuse angeordnet, dass die Kartusche von einer geschlossenen Stellung, in der die Abströmöffnung vollständig vom Gehäuse bedeckt ist, in eine Stellung beweglich ist, in der die Durchströmöffnung über der Abströmöffnung liegt und somit eine Abgabe des Duftstoffs von dem in der Kartusche enthaltenen Duftstoffträger in den Fahrzeuginnenraum ermöglicht. Über Markierungen kann der Fahrzeuginsasse die Stellung der Kartusche und der Abströmöffnungen sicher erkennen.

In der DE 90 05 101 U1 ist ein dosierbares Duftstoffgerät zur Anwendung in einem Personenkraftfahrzeug beschrieben, dass ein einfaches Auswechseln des Duftstoffs nach dem Verbrauch ermöglicht. Das Duftstoffgerät besteht aus einer mit Perforationen versehenen Kapsel, in die der Duftstoff einbringbar ist. Die Kapsel ist zweiteilig, wobei die beiden Teile mittels eines Scharnieres oder dergleichen verbunden sind. Der eine Teil der Kapsel weist eine ebene Rückwand auf, auf der zur haftenden Anbringung der Kapsel ein Klebestreifen angeordnet ist. Der andere Teil der Kapsel ist mit den Perforationen versehen und ist mittels einer Raste mit dem einen Teil verbindbar.

Weitere dosierbare Duftstoffgeräte zur Anwendung in einem Personenkraftfahrzeug sind beispielsweise in der DE 297 19 510 U1 und DE 298 12 056 U1 beschrieben.

Aufgabe der vorliegenden Erfindung ist es, ein dosierbares Duftstoffgerät der eingangs genannten Art so weiter zu bilden, dass bei einfacher Bauweise und unkomplizierter Handhabung des Geräts eine dosierte Duftstoffabgabe gewährleistet ist und überdies die Möglichkeit besteht, das Gerät mit Duftstoff nachzufüllen, den Duftstoff auszutauschen oder zu entfernen.

Gelöst wird die Aufgabe bei dem dosierbaren Duftstoffgerät der eingangs genannten Art dadurch, dass das Gehäuse die Form einer Kugel besitzt, die die Dufteintrittsöffnung, die Luftaustrittsöffnung und die Nachfüllöffnung für den Duftstoff aufweist, wobei die Öffnungen mittels der Abdeckung geöffnet und verschlossen werden können, wobei die Abdeckung kreisbogenförmig ausgebildet ist, als länglicher, sich über einen Kreibogen erstreckender Streifen.

Die Ausbildung des Duftstoffgerätes mit Lufteintrittsöffnung und Luftaustrittsöffnung stellt sicher, dass das Gehäuse von der Luft durchströmt wird und demzufolge der Duftstoff in Abhängigkeit von dem Luftdurchsatz durch das Gehäuse mehr oder weniger Intensiv aus dem Gehäuse austritt.

Die Intensität wird durch die Abdeckung geregelt, indem mittels dieser der Öffnungsquerschnitt von Lufteintrittsöffnung und Luftaustrittsöffnung variabel bemessen werden kann.

Bevorzugt weist das Gehäuse eine einzige Lufteintrittsöffnung und mehrere Luftaustrittsöffnungen auf. Zweckmäßig sind diese Öffnungen derart angeordnet, dass beim Verstellen der Abdeckung, wobei dies insbesondere eine Verschiebebewegung der Abdeckung relativ zum Gehäuse ist, zunächst die Lufteintrittsöffnung, anschließend die Luftaustrittsöffnung bzw. nacheinander die diversen Luftaustrittsöffnungen bei geöffneter Lufteintrittsöffnung freigegeben werden. Zum Öffnen der Nachfüllöffnung, die vorzugsweise wesentlich größer ist als die Lufteintrittsöffnung bzw. die Luftaustrittsöffnung, wird die Abdeckung vorzugsweise vom Gehäuse entfernt. Um die Abdeckung auf einfache Art und Weise vom Gehäuse lösen zu können, ist die Abdeckung vorzugsweise mittels einer Klipsverbindung mit dem Gehäuse verbunden.

Bei der erfindungsgemäßen Gestaltung des Gehäuses in Form der Kugel, wird es als bevorzugt angesehen, wenn die Abdeckung außerhalb des Gehäuses angeordnet ist. Die Abdeckung kann auf der Außenseite einen nach innen gerichteten Rücksprung zur Aufnahme des Streifens aufweisen, der somit im Gehäuse geführt ist. Es wird als bevorzugt angesehen, wenn die Abdeckung sich über einen Sektor von mehr als 180°, insbesondere einen Sektor von 200° bis 220° erstreckt. Dies ermöglicht es, die streifenförmige Abdeckung mittels Überdehnung auf das Gehäuse aufzustecken. Um die Abdeckung einfach bezüglich des Gehäuses verschieben zu können, ist diese vorzugsweise auf ihrer Außenseite mit einer Griffmulde versehen.

Die Lagerung des Gehäuses erfolgt auf besonders einfache Art und Weise und bei einfacher konstruktiver Gestaltung, indem das Gehäuse in einer Ringblende gehalten ist, insbesondere in einer geklipsten Ringblende gehalten ist. Die Ringblende ist beispielsweise in der Armaturentafel des Fahrzeugs oder in einer weiteren Blende des Fahrzeugs gehalten. Auch hier erfolgt die Lagerung insbesondere durch eine Klipsverbindung.

Die Ringblende ist vorzugsweise mit einer umlaufenden Ringdichtung zum Abdichten des Gehäuses versehen.

Das erfindungsgemäße Duftstoffgerät gestattet es, den ausströmenden Duftstoff zu dosieren. Der Duftstoff kann durch die Nachfüllöffnung nachgefüllt, ausgetauscht oder entfernt werden. Das Duftstoffgerät ist im Fahrzeug so integriert, dass keine anderen Bauteile beeinträchtigt werden, z. B. das zum Stand der Technik beschriebene Lüftungsgitter.

Bevorzugte Weiterbildungen der Erfindung ergeben sich aus den Unteransprüchen und der nachfolgenden Beschreibung.

Nachfolgend wird ein Ausführungsbeispiel der Erfindung, ohne hierauf beschränkt zu sein, unter Bezugnahme auf die Zeichnung näher erläutert. In der Zeichnung zeigt:
- Figur 1: eine Ansicht des erfindungsgemäßen dosierbaren Duftstoffgeräts,
- Figur 2: ein Schnitt, durch das in Figur 1 gezeigte Duftstoffgerät, geschnitten gemäß der Linie A-A in Figur 1, bei die Öffnungen des Gehäuses verschließender Abdeckung,
- Figur 3: ein Schnitt gemäß Figur 2, bei die Lüfteintrittsöffnung und einige der Luftaustrittsöffnungen freigebender Abdeckung.

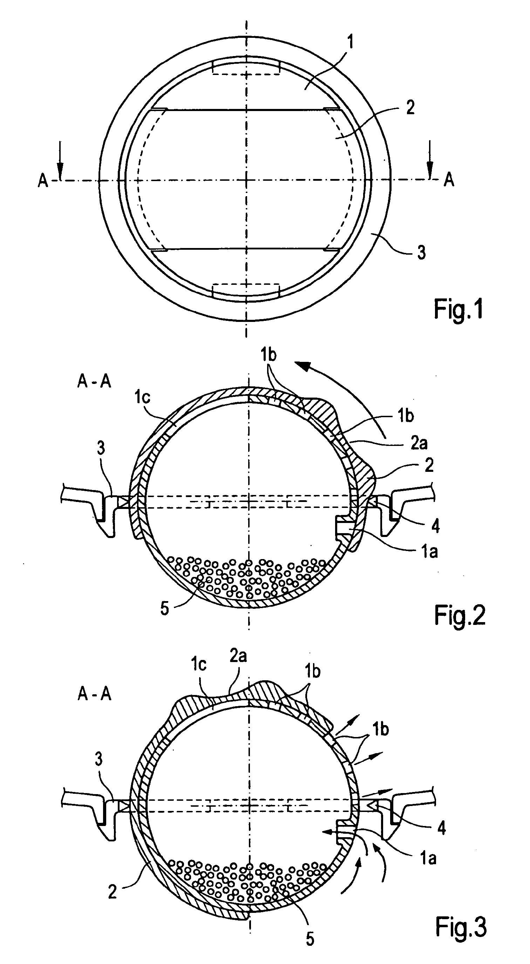

Das in den Figuren 1 bis 3 veranschaulichte Duftstoffgerät kann sowohl in einem luftumströmten Bereich, als auch in einem nicht luftumströmten Bereich eingesetzt werden, je nach Form und Art des Duftstoffes.

Das Gehäuse 1, das die Funktion eines Reservoirs hat, besitzt die Form einer Kugel und ist mit einer Lufteintrittsöffnung 1a mit kreisrundem Querschnitt sowie fünf Luftaustrittsöffnungen 1b in Form parallel zueinander angeordneter Schlitze und einer großen Nachfüllöffnung 1c versehen. Die Öffnungen 1a und 1b können mittels Verstellung der verschiebbaren Abdeckung 2 geöffnet werden. Diese ist außerhalb des Gehäuses 1 angeordnet und kreisbogenförmig ausgebildet. Konkret ist die Abdeckung 2 als länglicher, sich über einen Kreisbogensektor von 210° erstreckender Steifen ausgebildet.

Beim Verstellen der verschiebbaren Abdeckung 2, zum Öffnen der Öffnungen, wird, ausgehend von der Verschlussstellung gemäß Figur 2, zuerst die Öffnung 1a geöffnet und Luft strömt in das Gehäuse 1. Beim weiteren Verschieben der Abdeckung 2 werden nachfolgend mehrere Luftaustrittsöffnungen 1b frei, sodass eine Dosierung mit Duftstoff angereicherter Luft möglich ist. Das Gehäuse 1 ist geklipst mit einer Ringblende 3, die in der Armaturentafel des Fahrzeuges oder in einer weiteren Blende mittels Klipsen befestigt ist. Die Ringblende 3 besitzt eine umlaufende Gummidichtung 4, um Luftaustritt zu vermeiden. Die Abdeckung 2 besitzt eine Griffmulde 2a zur Verstellung und wird auf das Gehäuse 1 mittels Überdehnung aufgesteckt, ähnlich einem Kugelgelenk. Durch Abziehen der Abdeckung 2 kann Duftstoff 5 durch die Nachfüllöffnung 1c nachgefüllt werden. Um das Gehäuse 1 zu entleeren, kann es von der Ringblende 3 gelöst werden.

### Bezugszeichenliste

- Gehäuse: 1
- Lufteintrittsöffnung: 1a
- Luftaustrittsöffnung: 1b
- Nachfüllöffnung: 1c
- Abdeckung: 2
- Griffmulde: 2a
- Ringblende: 3
- Gummidichtung: 4
- Duftstoff: 5

## Patentansprüche

1. Dosierbares Duftstoffgerät zur Anwendung in einem Kraftfahrzeug, insbesondere einem Personenkraftfahrzeug, mit einem im Fahrzeuginneren angeordneten Gehäuse (1) zur Aufnahme eines Duftstoffs (5), wobei das Gehäuse (1) mit mindestens einer Lufteintrittsöffnung (1a) und mindestens einer Luftaustrittsöffnung (1b) sowie einer Nachfüllöffnung (1c) für den Duftstoff versehen ist, und die mindestens eine Lufteintrittsöffung (1a) und die mindestens eine Luftaustrittsöffnung (1b) durch Verstellen einer Abdeckung (2) geöffnet und verschlossen werden kann, **dadurch gekennzeichnet, dass** das Gehäuse (1) die Form einer Kugel besitzt, die die Lufteintrittsöffnung (1a), die Luftaustrittsöffnung (1b) und die Nachfüllöffnung (1c) für den Duftstoff (5) aufweist, wobei die Öffnungen (1a, 1b, 1c) mittels der Abdeckung (2) geöffnet und geschlossen werden können. wobei die Abdeckung (2) kreisbogenförmig ausgebildet ist, als länglicher, sich über einen Kreisbogen erstreckender Streifen.

2. Gerät nach Anspruch 1, **dadurch gekennzeichnet, dass** das Gehäuse (1) eine einzige Lufteintrittsöffnung (1a) und/oder mehrere Luftaustrittsöffnungen (1b) besitzt.

3. Gerät nach Anspruch 1 oder 2, **dadurch gekennzeichnet, dass** die jeweilige
Luftaustrittsöffnung (1b) als Schlitz ausgebildet ist.

4. Gerät nach einem der Ansprüche 1 bis 3, **dadurch gekennzeichnet, dass** die Abdeckung (2) außerhalb des Gehäuses (1) angeordnet ist.

5. Gerät nach einem der Ansprüche 1 bis 4, **dadurch gekennzeichnet, dass** die Abdeckung (2) verschiebbar im Gehäuse (1) gelagert ist.

6. Gerät nach einem der Ansprüche 1 bis 5, **dadurch gekennzeichnet, dass** die Abdeckung (2) lösbar mit dem Gehäuse (1) verbunden ist, insbesondere mittels einer Klipsverbindung mit dem Gehäuse (1) verbunden ist.

7. Gerät nach einem der Ansprüche 1 bis 6, **dadurch gekennzeichnet, dass** die Abdeckungen (2) sich über einen Sektor von mehr als 180°, insbesondere über einen Sektor von 200° bis 220° erstrecken.

8. Gerät nach einem der Ansprüche 1 bis 7, **dadurch gekennzeichnet, dass** die Abdeckung (2) an ihrer Außenseite mit einer Griffmulde (2a) versehen ist.

9. Gerät nach einem der Ansprüche 1 bis 8, **dadurch gekennzeichnet, dass** die Öffnungen (1a, 1b, 1c) derart angeordnet sind, dass beim Verschieben der Abdeckung (2) relativ zum Gehäuse (1), zunächst die Lufteintrittsöffnung (1a), anschließend die Luftaustrittsöffnung (2b) bzw. nacheinander die die diversen Luftaustrittsöffnungen (2b), bei geöffneter Lufteintrittsöffnung (1a), freigegeben werden.

10. Gerät nach einem der Ansprüche 1 bis 9, **dadurch gekennzeichnet, dass** zum Öffnen der Nachfüllöffnung (1c) die Abdeckung (2) vom Gehäuse (1) entfernt wird.

11. Gerät nach einem der Ansprüche 1 bis 10, **dadurch gekennzeichnet, dass** das Gehäuse (1) in einer Ringblende (3) gehalten ist, insbesondere geklipst in der Ringblende (3) gehalten ist.

12. Gerät nach Anspruch 11, **dadurch gekennzeichnet, dass** die Ringblende (3) in der Armaturentafel des Fahrzeugs oder in einer weiteren Blende des Fahrzeugs gehalten ist, insbesondere durch Klipsen gehalten ist.

13. Gerät nach Anspruch 11 oder 12, **dadurch gekennzeichnet, dass** die Ringblende (3) eine umlaufende Ringdichtung (4) zum Abdichten des Gehäuses (1) aufweist.

## Claims

1. A dosable fragrance device for use in a motor vehicle, especially a passenger car, comprising a housing (1) arranged in the interior of a vehicle for receiving a fragrance (5), with the housing (1) being provided with at least one air inlet opening (1a) and at least one air outlet opening (1b) and a refilling opening (1c) for the fragrance, and the at least one air inlet opening (1a) and the at least one air outlet opening (1b) can be opened and closed by adjusting a cover (2), **characterized in that** the housing (1) has the shape of a ball comprising the air inlet opening (1a), the air outlet opening (1b) and the refilling opening (1c) for the fragrance (5), with the openings (1a, 1b, 1c) being openable and closable by means of the cover (2), with the cover (2) being arranged in the manner of a circular arc as an oblong strip extending over the arc of a circle.

2. A device according to claim 1, **characterized in that** the housing (1) comprises a single air inlet opening (1a) and/or several air outlet openings (1b).

3. A device according to claim 1 or 2, **characterized in that** the respective air outlet opening (1b) is arranged as a slit.

4. A device according to one of the claims 1 to 3, **characterized in that** the cover (2) is arranged outside of the housing (1).

5. A device according to one of the claims 1 to 4, **characterized in that** the cover (2) is held in a displaceable manner in the housing (1).

6. A device according to one of the claims 1 to 5, **characterized in that** the cover (2) is detachably connected with the housing (1), especially connected with the housing by means of a clip-on connection.

7. A device according to one of the claims 1 to 6, **characterized in that** covers (2) extend over a sector of more than 180°, especially over a sector of 200° to 220°.

8. A device according to one of the claims 1 to 7, **characterized in that** the cover (2) is provided on its outside with a recessed grip (2a).

9. A device according to one of the claims 1 to 8, **characterized in that** the openings (1a, 1b, 1c) are arranged in such a way that upon displacement of the cover (2) relative to the housing (1) at first the air inlet opening (1a) is released, subsequently the air outlet opening (2b) or successively the various air outlet openings (2b), when the air inlet opening (1a) is opened.

10. A device according to one of the claims 1 to 9, **characterized in that** the cover (2) is removed from the housing (1) for opening the refilling opening (1c).

11. A device according to one of the claims 1 to 10, **characterized in that** the housing (1) is held in a circular orifice (3), especially held in a clipped manner in the circular orifice (3).

12. A device according to claim 11, **characterized in that** the circular orifice (3) is held in the dashboard of the vehicle or in a further panel of the vehicle, especially held by a clip-on connection.

13. A device according to claim 11 or 12, **characterized in that** the circular orifice (3) comprises a circumferential annular seal (4) for sealing the housing (1).

## Revendications

1. Appareil diffuseur de parfum dosable destiné à être utilisé dans un véhicule à moteur, en particulier un véhicule personnel, avec un boîtier (1) disposé à l'intérieur du véhicule et destiné à contenir un parfum (5), lequel boîtier (1) est muni d'au moins une ouverture d'entrée d'air (1a) et d'au moins une ouverture de sortie d'air (1b) ainsi que d'une ouverture de remplissage (1c) pour le parfum, et l'au moins une ouverture d'entrée d'air (1a) et l'au moins une ouverture de sortie d'air (1b) peuvent être fermées et ouvertes par le déplacement d'un couvercle (2), **caractérisé en ce que** le boîtier (1) a la forme d'une sphère qui présente l'ouverture d'entrée d'air (1a), l'ouverture de sortie d'air (1b) et l'ouverture de remplissage (1c) pour le parfum (5), les ouvertures (1a, 1b, 1c) pouvant être ouvertes et fermées au moyen du couvercle (2), lequel couvercle (2) est conformé en arc de cercle sous la forme d'une bande allongée s'étendant sur un arc de cercle.

2. Appareil selon la revendication 1, **caractérisé en ce que** le boîtier (1) possède une seule ouverture d'entrée d'air (1a) et/ou plusieurs ouvertures de sortie d'air (1b).

3. Appareil selon la revendication 1 ou 2, **caractérisé en ce que** chaque ouverture de sortie d'air (1b) est conformée comme une fente.

4. Appareil selon l'une des revendications 1 à 3, **caractérisé en ce que** le couvercle (2) est disposé à l'extérieur de l'appareil (1).

5. Appareil selon l'une des revendications 1 à 4, **caractérisé en ce que** le couvercle (2) est supporté de façon coulissante dans le boîtier (1).

6. Appareil selon l'une des revendications 1 à 5, **caractérisé en ce que** le couvercle (2) est relié au couvercle (1) de façon amovible, en particulier relié au boîtier (1) au moyen d'un assemblage clipsé.

7. Appareil selon l'une des revendications 1 à 6, **caractérisé en ce que** les couvercles (2) s'étendent sur un secteur de plus de 180°, en particulier sur un secteur de 200° à 220°.

8. Appareil selon l'une des revendications 1 à 7, **caractérisé en ce que** le couvercle (2) est muni sur sa face extérieure d'un creux de préhension (2a).

9. Appareil selon l'une des revendications 1 à 8, **caractérisé en ce que** les ouvertures (1a, 1b, 1c) sont disposées de telle sorte que lors d'une translation du couvercle (2) par rapport au boîtier (1), l'ouverture d'entrée d'air (1a) est d'abord dégagée, puis l'ouverture de sortie d'air (2b) ou les différentes ouvertures de sortie d'air (2b) l'une après l'autre, quand l'ouverture d'entrée d'air (1a) est ouverte.

10. Appareil selon l'une des revendications 1 à 9, **caractérisé en ce que** pour ouvrir l'ouverture de remplissage (1c), le couvercle (2) est enlevé du boîtier (1).

11. Appareil selon l'une des revendications 1 à 10, **caractérisé en ce que** le boîtier (1) est retenu dans un panneau annulaire (3), en particulier clipsé dans le panneau annulaire (3).

12. Appareil selon la revendication 11, **caractérisé en ce que** le panneau annulaire (3) est retenu dans le tableau de bord du véhicule ou un autre panneau du véhicule, en particulier retenu par des clips.

13. Appareil selon la revendication 11 ou 12, **caractérisé en ce que** le panneau annulaire (3) présente un joint annulaire (4) continu pour l'étanchéité du boîtier (1).
